# EUROPEAN PATENT APPLICATION

(11) **EP 0 638 648 A2**
(43) Date of publication of application: **15.02.1995**
(21) Application number: 94305815.6
(22) Date of filing: 05.08.1994
(51) Int. Cl.: C12P 21/08, C12N 5/00

(54) **Monoclonal antibodies specifically reacting with PAF receptor, production process thereof, and hybridomas producing the antibodies**

(30) Priority: 09.08.1993 JP 217003/93
(71) Applicant: SUMITOMO ELECTRIC INDUSTRIES, LIMITED, Osaka 541 (JP)
(72) Inventor: Shimizu, Takao, Bunkyo-ku, Tokyo (JP); Miyabe, Yuki, c/o Yokohama Works of, Yokohama-shi, Kanagawa-ken (JP); Nakata, Motomi, c/o Yokohama Works of, Yokohama-shi, Kanagawa-ken (JP)
(74) Representative: Perry, Robert Edward

(57) **Abstract**

Monoclonal antibodies specifically reacting to a PAF receptor which is a cell surface molecule are provided.

A process for producing monoclonal antibodies specifically reacting with a PAF receptor, which comprises the steps of (1) immunizing a rodent with a peptide of a PAF receptor, (2) taking out the spleen of the immune rodent to form a suspension of splenocytes, (3) mixing the suspension of the splenocytes with myeloma cells of a mouse in a hybridization accelerator to fuse both cells, (4) diluting the fused cells with a medium which does not favor unfused myeloma cells to culture the fused cells, thereby sorting hybridomas produced by the fusion of the antibody-producing cells with the myeloma cells, (5) confirming the presence of an antibody in a supernatant in each of culture wells separately containing the hybridomas using, as an indicator, the reactivity to the PAF receptor peptide, (6) selecting hybridomas which produce the desired antibodies and then cloning the hybridomas by the limiting dilution technique, and (7) recovering the desired antibodies from the culture supernatants of the monoclonal hybridoma cells is also provided.

Hybridomas separately producing monoclonal antibodies which react specifically with a PAF receptor are further provided.

## Description

### FIELD OF THE INVENTION

The present invention relates to monoclonal antibodies, and more particularly to monoclonal antibodies specifically reacting with a platelet-activating factor receptor (PAF-R) which is a cell surface molecule, a process for producing the monoclonal antibodies, and hybridomas producing the monoclonal antibodies.

### BACKGROUND OF THE INVENTION

A platelet-activating factor (PAF) is a phospholipid found as a factor derived from a leukocyte and inducing the activation of platelets, for example, shape change, histamine release and aggregation, and has a structure of 1-O-alkyl-2-acetyl-sn-glycero-3-phosphocholine.

PAF is a phospholipid autacoid produced in platelets, leukocytes, lung, spleen, kidney, brain, etc., has potent inflaming effect and hypotensive effect in a small amount, and is considered to participate in bronchial asthma, endotoxin shock, anaphylactic shock and the like. More specifically, PAF has not only a platelet-activating effect, but also neutrophil-activating effect, macrophage-activating effect, hypotensive effect, vascular permeability-accelerating effect, smooth muscle-contracting effect, glycogenolysis-accelerating effect in the liver, and the like, and is a factor involved in the development of anaphylaxis, inflammation, allergy and the like in vivo. PAF activates a target cell such as a platelet, neutrophil or macrophage through a receptor on a cell membrane.

The presence of this physiologically active phospholipid was confirmed in 1972, and its structure was clarified in 1979. However, the analysis of the PAF receptor has not been very developed up to date. The reasons are considered, for example, to be as follows. Since PAF is lipophilic and high in nonspecific affinity for cell membranes, even a basic ligand binding experiment is difficult to conduct. Besides, since a receptor protein exists only in a small amount in a cell membrane, its purification is difficult.

In 1991, Shimizu who is a coinventor of the present invention, et al. succeeded in isolating cDNAs of the PAF receptor from the guinea pig lung and human leukocytes using a genetic engineering technique (Nature, Vol. 349, 342, 1991). From the structure of the PAF receptor presumed from these two cDNAs and expression experiments, knowledge as to the functions of PAF and PAF receptor, and the pathogenic mechanisms of morbid states associated with PAF is on the point of being obtained.

However, the analysis of the PAF receptor at the protein level is not developed under the circumstances because a gene for the PAF receptor has been just obtained, and the PAF receptor can not hence be produced by a genetic engineering technique, so that great difficulties are encountered on acquirement of proteins. With respect to antibodies against the PAF receptor, there has also not been obtained in the present situation any polyclonal antiserum, to say nothing of monoclonal antibodies, so that their analysis has not been advanced to the point of nothing.

### OBJECTS AND SUMMARY OF THE INVENTION

It is an object of the present invention to provide monoclonal antibodies specifically reacting with a PAF receptor, a production process thereof, and hybridomas producing the monoclonal antibodies.

The present inventors have considered that if a monoclonal antibody against the PAF receptor is produced under the above-described circumstances as to the analysis of the PAF receptor at the protein level, the analysis of antibodies against the PAF receptor and the like, the analyses as to reactions caused by the binding of the PAF and PAF receptor and the PAF receptor itself have been advanced, and hence carried out an extensive investigation. As a result, the present inventors have succeeded in acquiring monoclonal antibodies specifically reacting to the PAF receptor and hybridomas producing the monoclonal antibodies. The present invention has been led to completion on the basis of this finding.

According to the present invention, there are thus provided monoclonal antibodies specifically reacting with a PAF receptor which is a cell surface molecule.

According to the present invention, there is also provided a process for producing monoclonal antibodies specifically reacting with a PAF receptor, which comprises the steps of:
(1) immunizing a rodent with a peptide of a PAF receptor,
(2) taking out the spleen of the immune rodent to form a suspension of splenocytes,
(3) mixing the suspension of the splenocytes with myeloma cells of a mouse in a hybridization accelerator to fuse both cells,
(4) diluting the fused cells with a medium which does not favor unfused myeloma cells to culture the fused cells, thereby sorting hybridomas produced by the fusion of the antibody-producing cells with the myeloma cells,
(5) confirming the presence of an antibody in a supernatant in each of culture wells separately containing the hybridomas using, as an indicator, the reactivity to the PAF receptor peptide,
(6) selecting hybridomas which produce the desired antibodies and then cloning the hybridomas by the limiting dilution technique, and
(7) recovering the desired antibodies from the culture supernatants of the monoclonal hybridoma cells.

According to the present invention, there are further provided hybridomas separately producing monoclonal antibodies which react specifically with a PAF receptor.

Examples of the hybridomas according to the present invention include Hybridoma PAF-R14 deposited as FERM BP-4723 and YM-PAFR deposited as FERM BP-4722 in National Institute of Bioscience and Human-Technology, Agency of Industrial Science and Technology, Japan, on the basis of Budapest Treaty on the International Recognition of the Deposit of Microorganisms for the Purposes of Patent Procedure.

### BRIEF DESCRIPTION OF THE DRAWINGS

FIG. 1 is an FACS diagram illustrating that an antibody produced by a hybridoma according to the present invention reacts with cells on which PAF-R molecules have been expressed. A dotted curve represents a negative control.

FIG. 2 is an FACS diagram illustrating that an antibody produced by another hybridoma according to the present invention reacts with cells on which PAF-R molecules have been expressed. A dotted curve represents a negative control.

FIG. 3 is an FACS diagram illustrating that an antibody produced by a further hybridoma according to the present invention reacts with cells on which PAF-R molecules have been expressed. A dotted curve represents a negative control.

FIG. 4 is an FACS diagram illustrating that an antibody produced by a still further hybridoma according to the present invention reacts with cells on which PAF-R molecules have been expressed. A dotted curve represents a negative control.

FIG. 5 is an FACS diagram illustrating that the antibody produced by the hybridoma according to the present invention reacts with eosinophils on which human PAF-R molecules have been expressed. A dotted curve represents a negative control.

FIG. 6 is an FACS diagram illustrating that the antibody produced by the hybridoma according to the present invention reacts with B cells (Raji) on which human PAF-R molecules have been expressed. A dotted curve represents a negative control.

FIG. 7 is an FACS diagram illustrating that the antibody produced by the hybridoma according to the present invention reacts with monocytes (U937) on which human PAF-R molecules have been expressed. A dotted curve represents a negative control.

FIG. 8 is an FACS diagram illustrating that the antibody produced by the hybridoma according to the present invention does not react with T cells (Molt-4) on which human PAF-R molecules have been expressed. A dotted curve represents a negative control.

FIG. 9 illustrates the results of an immuno-precipitation.

### DETAILED DESCRIPTION OF THE INVENTION

The present invention will hereinafter be described in detail.

Monoclonal antibodies specifically reacting with a PAF receptor, and hybridomas producing these monoclonal antibodies can be produced in accordance with the following procedure.
(1) A rodent is immunized with a peptide of a PAF receptor. More specifically, a peptide of a PAF receptor molecule of a guinea pig is synthesized. A carrier protein is coupled to this peptide to prepare a carrier-hapten complex. This complex is administered as an immunogen to the rodent to immunize the rodent. A Balb/c mouse or the like is used as the rodent.
(2) Antibody-producing cells are prepared from the immune rodent. More specifically, the spleen is taken out of the immune rodent to form a suspension of splenocytes.
(3) The suspension of the splenocytes is mixed with myeloma cells of a mouse in a hybridization accelerator to fuse both cells. As the myeloma cells, are used those distinguishable from the antibody-producing cells in a subsequent selective culture. Such myeloma cells may include myeloma cell strains such as P3U1. Polyethylene glycol (PEG) is a typical hybridization accelerator.
(4) The fused cells are diluted with a medium which does not favor unfused myeloma cells to culture the fused cells. More specifically, the fuses cells are cultured in a selective medium in which the antibody-producing cells are viable, but the myeloma cells are killed, thereby sorting hybridomas produced by the fusion of the antibody-producing cells with the myeloma cells. For example, if a 8-azaguanine-resistant myeloma cell strain is used, a hypoxanthine-aminopterine-thymidine containing medium (HAT medium) is used.
(5) The presence of an antibody in a supernatant in each of culture wells separately containing the hybridomas is confirmed using, as an indicator, the reactivity to the PAF receptor peptide. More specifically, whether the antibody secreted in the culture supernatant of cells proliferated in the selective medium is an antibody against the desired antigen is determined by whether the antibody reacts with the PAF receptor molecule or not. Hybridomas which produce the desired antibodies are then selected.
(6) After the selection of the hybridomas which produces the desired antibodies, the hybridomas are cloned by the limiting dilution technique. More specifically, cells in the culture wells in which cells secreting the desired antibodies exist are cloned by the limiting dilution technique.
(7) The desired antibodies are recovered from the culture supernatants of the monoclonal hybridoma cells. More specifically, a clone from which the desired antibody is secreted is selected to conduct cloning again, thereby establishing a clone of the hybridoma which secretes the monoclonal antibody against the desired antigen. The monoclonal antibody is prepared from the culture supernatant of this hybridoma.

The monoclonal antibody specifically reacting with the PAF receptor reacts with a peptide of the extracellular third or forth domain, or the intracellular fourth domain of the PAF receptor molecule.

More specifically, the PAF receptor is composed of 342 amino acid residues, and the molecular weight estimated from its primary structure is 38,982. Hydropathy analysis of the PAF receptor revealed that the PAF receptor has seven lipophilic domains, and it is hence considered to have a seven-times transmembrane structure (Biochemical Pharmacology, Vol. 44, No. 6, 1001-1008, 1992). The PAF receptor has 4 domains in its extracellular space, and the second, third and fourth domains thereof each have a loop-type structure. Besides, the PAF receptor has 4 domains in its intracellular space, and the first, second and third domains thereof each have a loop-type structure.

When respective peptides of the extracellular third and forth domains, and the intracellular fourth domain of the PAF receptor molecule of a guinea pig are synthesized and administered as immunogens to a rodent to sensitize the rodent, monoclonal antibodies reacting with the respective peptides of these domains can be obtained by the above-described procedure.

The monoclonal antibodies according to the present invention are preferably those reacting with peptides of the extracellular third domain of the PAF receptor. As such monoclonal antibodies, four monoclonal antibodies have been found and are classified into 3 antibodies belonging to a class of IgM and having a κ-L chain, and an antibody belonging to a class of IgG and a subclass of IgG2a and having a κ-L chain.

The monoclonal antibodies according to the present invention react with human eosinophils on which human PAF-R molecules have been expressed. The monoclonal antibodies according to the present invention also react with human B cells and monocytes, but do not react with human T cells.

The monoclonal antibodies according to the present invention react with proteins of about 40 kDa in CHO-PR1 and CHO-WT6'A which are cells transfected with a PAF-R gene, and Raji and Wa which are B cells.

The present invention includes active fragments of the monoclonal antibodies specifically reacting with the PAF receptor. The monoclonal antibodies according to the present invention are useful for immunological researches as to many reactions caused by the binding of PAF and PAF-receptor, immunotherapy, immunodiagnosis, and the like. It is not always necessary to use the whole monoclonal antibody molecule for such applications. Its active fragment may be used. As easily understood by those skilled in the art, the use of the active fragment may be preferred in some cases.

The monoclonal antibodies are homogeneous immunoglobulins recognizing a specific antigenic substance. The term "active fragment" means a fragment of a monoclonal antibody active in antigen-antibody reaction. As specific examples thereof, may be mentioned F(ab')₂, Fab', Fab, Fv and recombinant Fv. These active fragments may be prepared from the monoclonal antibodies according to the present invention in accordance with a method known per se in the art.

The F(ab')₂ fragment is one of fragments obtained by digesting an immunoglobulin IgG with pepsin. When IgG is digested with pepsin at a pH near 4.0, it is cleaved at a hinge area of its H chain to produce a fragment having a molecular weight of about 100,000. This cleavage takes place on the C-terminal side away from the disulfide bond between H chains. This fragment has two antigen-binding sites and hence can couple to an antigen, thereby undergoing precipitin reaction and agglutination reaction.

The Fab' fragment is a fragment produced by reducing the F(ab')₂ fragment with a reagent such as 2-mercaptoethanol and alkylating the reduced product with monoiodoacetic acid, thereby cleaving a disulfide bond between H chains, and having a molecular weight of about 50,000.

A Fab fragment (antigen-binding fragment) is one of fragments obtained by the papain digestion of IgG. When IgG is digested with papain in the presence of cysteine, its H chain is cleaved at a site on the N-terminal side away from the disulfide bond between H chains in a hinge area, thereby producing two Fab fragments and one Fc fragment (crystallizable fragment). The Fab fragment is a fragment in which a Fd fragment corresponding to about a half of the H chain on the N-terminal side (V_{H} domain + C_{H}1 dome is coupled to an L chain by a disulfide bond, said fragment having a molecular weight of about 45,000 and one antigen-binding site.

The Fv fragment is an antigen-binding fragment composed of a variable region of immunoglobulin heavy chain (V_{H}) and a variable region of immunoglobulin light chain (V_{L}), said variable regions being coupled to each other by a nonconjugate bond.

The recombinant Fv fragment can be obtained by sequencing a DNA from a hybridoma which produces a monoclonal antibody to determine base sequences which code V_{H} and L_{H}, respectively, and then integrating these DNA fragments in a vector to produce a monovalent active antibody fragment having a structure of V_{L}-Linker-V_{H}. In IgG, Fab or F(ab')₂, V_{H} and L_{H} are coupled to each other by an S-S bond. In the recombinant Fv fragment, a linker is inserted between V_{H} and L_{H} so as to take the same steric structure as the state coupled by the S-S bond. This fragment may be simply called "Fv" in some cases. It may also be called "SCFv (single chain FV)". The recombinant Fv fragment may also be expressed by microorganisms such as Escherichia coli and bacteriophages.

### ADVANTAGES OF THE INVENTION

The monoclonal antibodies according to the present invention against the PAF receptor are useful for explicating various reactions which are considered to be caused by the binding of PAF and PAF-R, and for understanding the mechanisms of PAF and PAF-R in bronchial asthma, endotoxin shock, anaphylactic shock and the like.

The monoclonal antibodies according to the present invention block the binding of PAF and PAF-R and hence are expected to prevent these various reactions.

Accordingly, the monoclonal antibodies, production process thereof and hybridomas producing these monoclonal antibodies according to the present invention are useful in fields of genetic engineering, medical science and protein engineering, and industrial fields associated with these fields.

### EMBODIMENTS OF THE INVENTION

The present invention will hereinafter be described in more detail by reference to the following examples.

### Example 1:

### 1. Synthesis of peptide:

Peptides of the extracellular third and forth domains, and the intracellular fourth domain of a guinea pig PAF receptor (PAF-R) were synthesized by a peptide synthesizer (Model 431A, manufactured by ABI Company), cleaved, purified by HPLC and then lyophilized.

The guinea pig PAF receptor is composed of 342 amino acid residues. The peptide of the extracellular third domain of the PAF receptor has an amino acid sequence of from the 158th to the 173th, Thr-Asn-Val-Val-Ser-Asn-Lys-Ala-Gly-Ser-Gly-Asn-Ile-Thr-Arg-Cys.

The peptide of the extracellular fourth domain of the guinea pig PAF receptor has a structure that Cys is added to the C-terminal of an amino acid sequence of from the 259th to the 273th, Glu-Leu-Gly-Met-Trp-Pro-Ser-Ser-Asn-His-Gln-Ala-Ile-Asn-Asp.

The peptide of the intracellular fourth domain of the guinea pig PAF receptor has a structure that Cys is added to the C-terminal of an amino acid sequence of from the 323th to the 337th, Asp-Thr-Gly-Thr-Glu-Met-Ala-IlePro-Ile-Asn-His-Thr-Pro-Val.

Each of the three peptides synthesized above was treated in accordance with the following procedures 2 to 6.

### 2. Immunization:

One milligram of the peptide synthesized above was dissolved in 200 µl of a conjugation buffer (product of Pierce Company). To the resulting solution, 500 µl of a carrier protein KLH (2 mg/ml) was added to mix them. The mixture was rotationally stirred overnight at 4°C to couple the peptide to KLH, thereby providing the thus- obtained complex as an immunogen.

The peptide-KLH complex in an amount of 10 µg in terms of the peptide was intraperitoneally injected into a Balb/c mouse together with FCA (Freund's complete adjuvant) in priming and with a poly A.poly U adjuvant on and after the second week. The peptide complex was injected once a week, 15 times in total, thereby immunizing the mouse.

### 3. Cell fusion:

Upon boosted time of 4 days after final immunization, the spleen was taken out of the mouse. The spleen was minced and filtered through a mesh and then suspended in an RPMI1640 medium, thereby obtaining 6 x 10⁷ splenocytes. The splenocytes and a mouse-derived 8-azaguanine-resistance myeloma cell strain (hypoxanthine-guanine phosphoribosyl transferase defective strain) P3U1 (1 x 10⁷ cells) were mixed with each other in a proportion of about 6:1, and the resulting mixture was centrifuged (1500 rpm, 5 minutes). To the thus-obtained cell pellets, 3 ml of a 50% solution of polyethylene glycol 4000 (product of Merck Company) in an RPMI1640 medium was added over 1 minute with stirring in hot water of 37°C. The resulting mixture was then stirred for 1.5 minutes, and was added with 10 ml of an RPMI1640 medium over 3 minutes with stirring, and further with 12 ml of an RPMI1640 medium over 1 minute with stirring, thereby conducting cell fusion.

After the cell fusion, a great amount (25 ml) of an HEPES-RPMI1640 solution was added, and the mixture was centrifuged (1500 rpm, 5 minutes) to remove a supernatant. On the resultant precipitate, 1 ml of a 10% FCS-RPM11640 medium containing hypoxanthine (100 µM), aminopterine (0.4 µM) and thymidine (10 µM) (HAT medium) was placed. After the HAT medium was left at rest for 30 minutes in a CO₂-incubator at 37°C, the splenocytes were adjusted to 1 x 10⁶ cells/ml in the incubator.

### 4. Selection of hybridoma:

The cell suspension prepared in the step 3 was poured in 200-µl portions into 3 microplates each having 96 wells to culture the cells in a CO₂-incubator controlled at 37°C and CO₂ concentration of 5%. After a week, it was confirmed that only hybridomas formed colonies and proliferated.

### 5. Detection of antibody (primary screening):

Since it was confirmed that the hybridomas fully proliferated, the culture supernatant of each well was used to detect antibodies reacting with the PAF-R peptide in the following manner.
(1) An antigen solution [PAF-R peptide (10 µg/ml)] was poured in portions of 50 µl/well into wells of an ELISA plate (Imuron 2, manufactured by Dynatec Company) with 96 wells, and the plate was allowed to stand overnight at 4°C.
(2) The antigen solution in each well was thrown out, and instead a 1% BSA/PBS solution was poured in a proportion of 200 µl/well to block it. The thus-treated plate was then left over for 2 hours at room temperature.
(3) The blocking solution was thrown out, and the culture supernatant was poured in an amount of 100 µl into the well, and the plate was left over for 1 hour at room temperature.
(4) The culture supernatant was thrown out, and the plate was washed 4 times with a 0.05% Tween 20/PBS solution. A biotinized anti-mouse IgG antibody (product of Vector Company) was poured in portions of 100 µl/well into the wells, and the plate was then left over for 1 hour at room temperature.
(5) The plate was washed 4 times with a 0.05% Tween 20/PBS solution, and an ABC solution (avidin-biotinized peroxidase combined solution, product of Vector Company) was poured in portions of 50 µl/well into the wells to conduct a reaction for 30 minutes.
(6) The plate was washed 4 times with a 0.05% Tween 20/PBS solution, and the hybridomas were stained with H₂O₂-OPD/PCB in an amount of 100 µl/well to measure respective A₄₉₀ values, thereby selecting wells containing antibodies high in reactivity to the peptide.

### 6. Cloning:

Hybridomas proliferated in the wells selected in the step 5 were separately cloned by the limiting dilution technique. The culture medium in each well was diluted with a 10% FCS-RPMI1640 medium so as to give a cell concentration of one cell/well. The thus-diluted culture medium was poured in portions of 200 µl/well into wells of a 96-well plate to culture the cell under conditions of 37°C and 5% CO₂. After about 1 week, the hybridomas were recognized to proliferate, and colonies became a certain size, and so the detection of antibodies was conducted again in accordance with the process of the step 5.

The above-described procedures 2 to 6 were repeated twice on the three peptides synthesized in the step 1, whereby 17 clones, which stably produced antibodies against PAF-R, could be obtained.

### 7. Staining of PAF-R molecule expressed cell by FACS:

Whether antibodies produced by the thus-obtained hybridomas react with cells which expressed a PAF-R molecule (cells transfected with a PAF-R gene), CHO-PR1, or not was determined by FACS (fluorescence activated cell sorter).

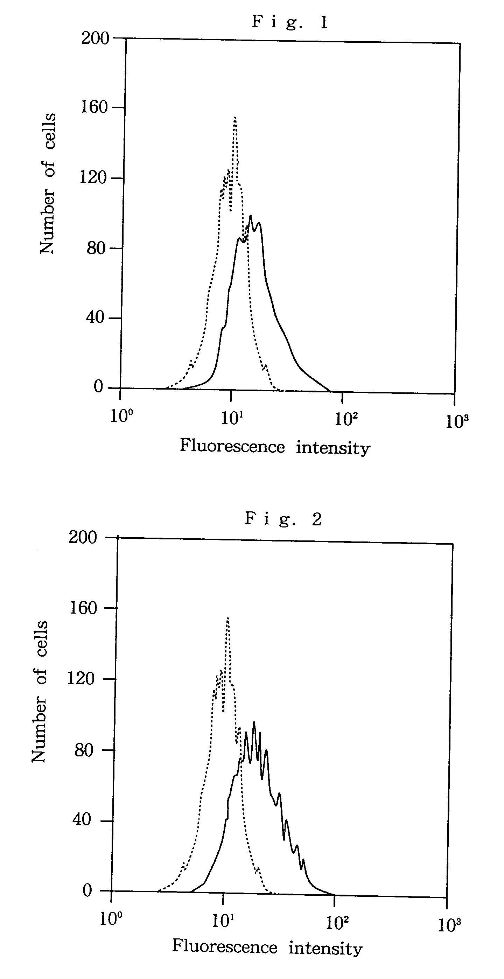

After collecting CHO-PR1, the cells were put in portions of 1 x 10⁶ cells into Fischer tubes, into which 200 µl of the culture supernatant was poured to react them for 20 minutes on an ice bath. Each of the reaction mixtures was centrifugally washed with PBS (3000 rpm, 1 minute, 3 times) and added with 100 µl of FITC-anti-hamster Ig's (product of Cultag Company, diluted to 1:100) to react them for 20 minutes on an ice bath. The reaction mixture was centrifugally washed twice with PBS to suspend it in 200 µl of PBS, thereby conducting measurement by FACScan. As a result, as illustrated in FIGs. 1 to 4, it was found that 4 antibodies reacting with the PAF-R molecule were obtained.

In each drawing, a histogram indicated by a dotted line represents a negative control free of a primary antibody. In each drawing, a histogram indicated by a continuous line was obtained by using, as a primary antibody, various monoclonal antibodies and, as a secondary antibody, a fluorescein (FITC)-labeled antibody commonly specific for all the primary antibodies. Accordingly, if the primary antibody reacts with a cell surface molecule, it becomes a cell population higher in fluorescence intensity than the cell population of the negative control, and so its graph shifts on the right of the graph of the negative control.

All the four antibodies react with the peptide of the extracellular third domain of PAF-R.

### 8. Determination of subclass of antibody:

The subclasses of antibodies produced by the resultant hybridomas were determined by enzyme immunoassay (EIA) in which anti-mouse antibodies (Anti-IgG1, IgG2a, IgG2b, IgG3, IgM, IgA) and anti-mouse L chain antibodies (anti-κ, λ) (MAB-isotyping kit, Pharmingen) were separately reacted with the anti-PAF-R monoclonal antibodies in the culture supernatants of the hybridomas. As a result, the resultant monoclonal antibodies were able to be classified into three antibodies in which staining was recognized in wells separately containing an anti-mouse antibody (anti-IgM) and an anti-mouse L chain antibody (anti-κ), and one antibody in which staining was recognized in wells separately containing an anti-mouse antibody (anti-IgG2a) and an anti-mouse L chain antibody (anti-κ). Namely, the three monoclonal antibodies belong to a class of IgM and have a κ-L chain, and the other one belongs to a class of IgG and a subclass of IgG2a and has a κ-L chain.

A hybridoma which produces a monoclonal antibody (FIG. 2; belonging to a class of IgM and having a κ-L chain) reacting with the peptide of the extracellular third domain of PAF-R is Hybridoma PAF-R14 deposited as FERM BP-4723 in National Institute of Bioscience and Human-Technology, Agency of Industrial Science and Technology, Japan.

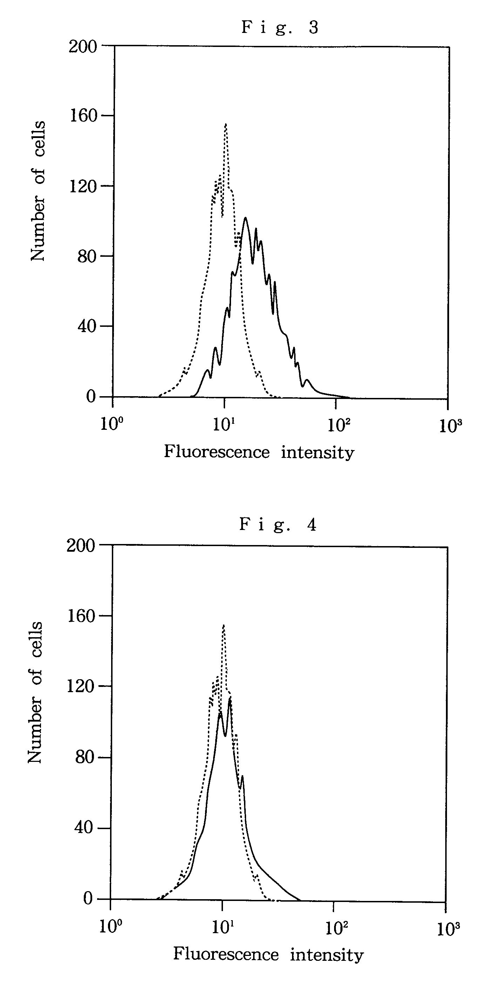

Besides, a hybridoma which produces a monoclonal antibody (FIG. 3; belonging to a subclass of IgG2a and having a κ-L chain) reacting with the peptide of the extracellular third domain of PAF-R is YM-PAFR deposited as FERM BP-4722 in National Institute of Bioscience and Human-Technology, Agency of Industrial Science and Technology, Japan.

### Example 2:

### 1. Staining of human eosinophil by FACS:

Whether the monoclonal antibody (FIG. 3; belonging to a subclass of IgG2a and having a κ-L chain) obtained in Example 1 reacts with human eosinophils which expressed a human PAF-R molecule, or not was determined by FACS.

After collecting human eosinophils, the cells were put in portions of 1 x 10⁶ cells into Fischer tubes, into which 50 µg/ml of the antibody purified was poured in an amount of 100 µl to react them for 20 minutes on an ice bath. Each of the reaction mixtures was centrifugally washed with PBS (3000 rpm, 1 minute, 3 times) and then added with 100 µl of FITC-anti-mouse Ig's (product of Caltag Company, diluted to 1:100) to react them for 20 minutes on an ice bath. The reaction mixture was centrifugally washed twice with PBS to suspend it in 200 µl of PBS, thereby conducting measurement by FACScan.

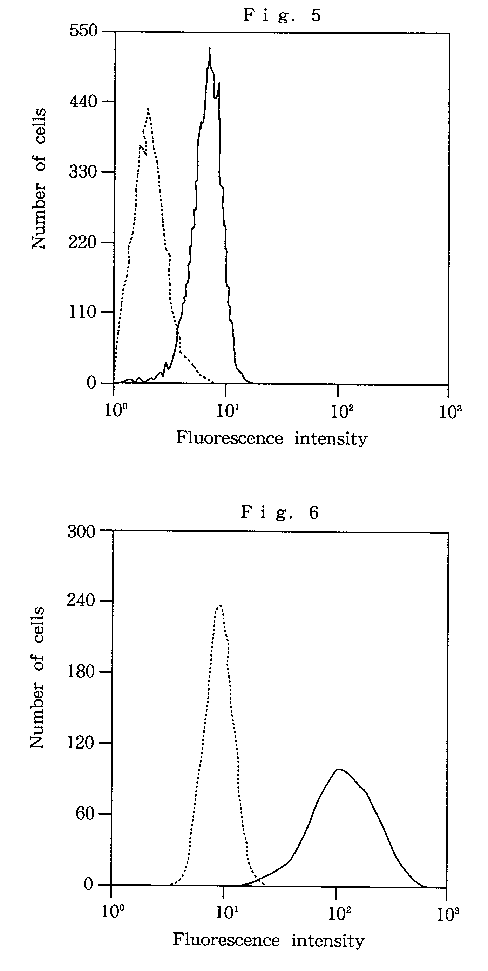

As a result, as illustrated in FIG. 5, it was revealed that the monoclonal antibody obtained in Example 1 also reacts with human PAF-R.

### 2. Staining of various human cell strains by FACS:

The respective cell strains of human B cells, T cells and monocytes were used to determine whether these cells express PAF-R or not in the following manner.

After collecting each cell strain, the cells were put in portions of 1 x 10⁶ cells into Fischer tubes, into which 10 µg/ml of the purified antibody obtained in Example 1 was poured in an amount of 100 µm to react them for 20 minutes on an ice bath. Each of the reaction mixtures was centrifugally washed with PBS (3000 rpm, 1 minute, 3 times) and then added with 100 µl of FITC-anti-mouse Ig's (product of Cultag Company, diluted to 1:100) to react them for 20 minutes on an ice bath. The reaction mixture was centrifugally washed twice with PBS to suspend it in 200 µl of PBS, thereby conducting measurement by FACScan.

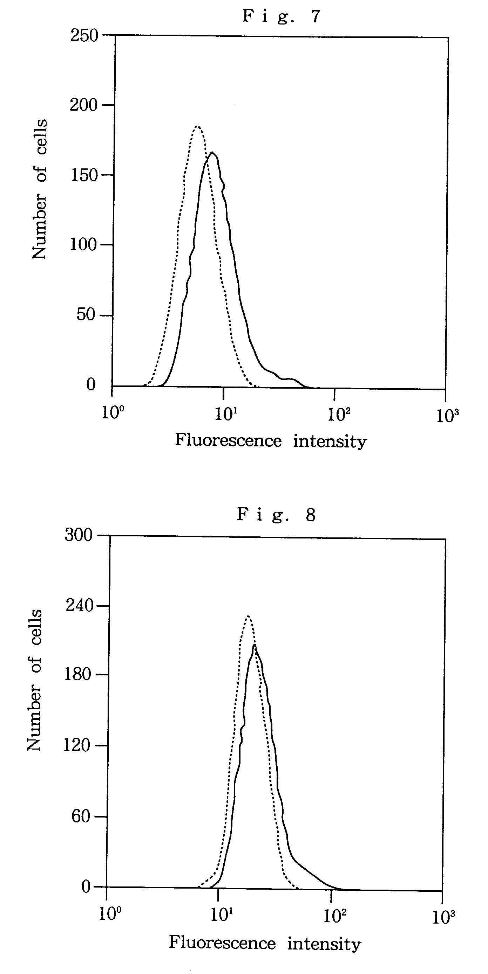

As a result, as illustrated in FIGs. 6 to 8, it was revealed that the monoclonal antibody obtained in Example 1 reacts with the B cells (Raji) and monocytes (U937), but does not react with the T cells (Molt-4). Therefore, it was found that PAF-R is expressed by the B cells and monocytes, but not expressed by the T cells.

### Example 3:

### Immunoprecipitation:

Two PAF-R gene transfected cells (CHO-PR1, CHO-WT6'A) and a parent strain (CHO) thereof as well as Raji, Wa, U937 and Molt-4 cells were separately cultured to 1 x 10⁷ cells using a 10% FCS-RPMI1640 medium in a 75-cm² culture flask. The respective cell samples were collected by centrifugation at 1500 rpm for 5 minutes and then centrifugally washed once with PBS. Thereafter, the cell samples were centrifugally washed 3 times with HBSS (Hanks' buffer solution).
(1) Biotylation:
   After sucking out each of the culture supernatants of the respective cell samples, the cultured cells were suspended in 2 ml of physiological saline containing a 0.1 M HEPES buffer solution (pH: 8.0). Thereafter, 1.0 mg/ml of sulfo NHS-biotin was placed in an amount of 20 µl in the suspension to conduct a reaction for 40 minutes at room temperature using a rotator. The reaction mixture was then centrifugally washed 3 times with an RPMI1640 solution cooled to 4°C.
(2) Cell lysis:
   Pellets of the thus-biotinized cells were added with 400 µl of a lytic buffer solution [1% NP-40, 20 mM Tris-HCl, 150 mM NaCl, 2 mM CaCl₂, 2 mM MgCl₂, 10 µM A-PMSF (phenylmethylsulfonyl fluoride), 50 µg/ml trypsin inhibitor], and the mixture was fully stirred and then left over for 30 minutes on an ice bath. Thereafter, the mixture was centrifuged for 10 minutes at 1500 rpm, and a supernatant was collected and divided into 200-µl portions.
(3) Preclear:
   Beads obtained by binding normal mouse IgG to CNBr-activated Sepharose gel (product of Pharmacia AB) were placed in an amount of 100 µl in 200 µl of the collected supernatant to conduct a reaction overnight at 4°C, thereby removing a nonspecific bound protein. Thereafter, gel was precipitated by centrifugation at 12000 rpm for 1 minute to collect a supernatant.
(4) Immunoprecipitation:
   In the supernatant collected, were placed 100 µm of beads obtained by binding the monoclonal antibody (FIG. 3; belonging to a subclass of IgG2a and having a κ-L chain) established in this invention to CNBr-activated Sepharose gel (product of Pharmacia AB) to conduct a reaction at 4°C for 2 hours. After the resultant gel was washed 3 times with a washing solution (0.5% NP-40, 20 mM Tris-HCl, 0.6 M NaCl), 20 µl of a sample buffer solution [10% glycerol, 5% 2-mercaptoethanol, 2% SDS, 0.625 M Tris-HCl (pH 6.8), 1 mg/100 ml of BPB (Bromophenol Blue)] containing 2% of SDS was put into the gel to boil them for 5 minutes. Thereafter, 10-20% of SDS-PAGE gel (product of Biocraft Company) was used to conduct electrophoresis. After the electrophoresis, proteins were transferred to a nitrocellulose membrane (product of Sartorius Company) using a transblot system (manufactured by Biorad Company).
(5) Development:
   The nitrocellulose membrane was immersed in Block Ace (product of Dainippon Pharmaceutical Co., Ltd.) and held for 1 hour therein to block it. Thereafter, it was immersed in an ABC solution (avidin-biotinized peroxidase complex solution, Vectastain ABC kit, product of Vector Company) to conduct a reaction for 30 minutes. The thus-treated nitrocellulose membrane was washed 3 times with a 0.05% Tween 20/PBS solution (incubated 3 times for 10 minutes). Thereafter, the proteins were caused to react using a stain kit of ECL Western Blotting Detection System manufactured by Amersham Company. Thereafter, exposure was performed with an X-ray film, whereby proteins of about 40 kDa, which react with the established antibody, were detected in CHO-PR1 and CHO-WT6'A which were cells transfected with a PAF-R gene, and Raji and Wa which were B cells.

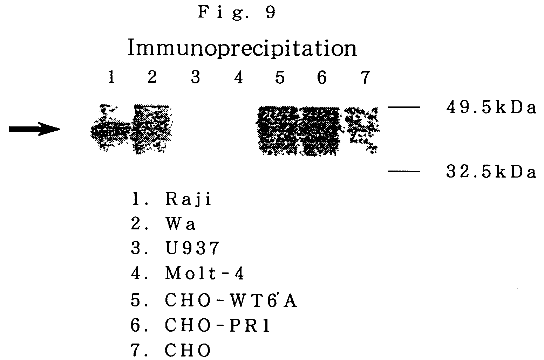

This result is illustrated in FIG. 9. Incidentally, the result of the immunoprecipitation was identified with the results of FACS, and it was hence confirmed that this antibody reacts with PAF-R, and a band detected is PAF-R.

## Claims

**1.** A monoclonal antibody specifically reacting with a PAF receptor which is a cell surface molecule.

**2.** The monoclonal antibody according to Claim 1, wherein a species of the PAF receptor is a guinea pig.

**3.** The monoclonal antibody according to Claim 1, which reacts with a peptide of the extracellular third or forth domain, or the intracellular fourth domain of the PAF receptor.

**4.** The monoclonal antibody according to Claim 1, which reacts with a peptide of the extracellular third domain of the PAF receptor.

**5.** The monoclonal antibody according to Claim 1, which belongs to a class of IgM and has a κ-L chain.

**6.** The monoclonal antibody according to Claim 1, which reacts with a peptide of the extracellular third domain of the PAF receptor, belongs to a class of IgM and has a κ-L chain.

**7.** The monoclonal antibody according to Claim 1, which belongs to a subclass of IgG2a and has a κ-L chain.

**8.** The monoclonal antibody according to Claim 1, which reacts with a peptide of the extracellular third domain of the PAF receptor, belongs to a subclass of IgG2a and has a κ-L chain.

**9.** The monoclonal antibody according to Claim 1, which reacts with human eosinophils which express a human PAF receptor molecule.

**10.** The monoclonal antibody according to Claim 1, which reacts with human B cells and monocytes which express a human PAF receptor molecule, but does not react with human T cells.

**11.** The monoclonal antibody according to Claim 1, which reacts with a peptide of the extracellular third domain of the PAF receptor, belongs to a subclass of IgG2a, has a κ-L chain, and reacts with human eosinophils, B cells and monocytes which express a human PAF receptor molecule, but does not react with human T cells.

**12.** The monoclonal antibody according to Claim 1, which reacts with proteins of about 40 kDa in CHO-PR1 and CHO-WT6'A which are cells transfected with a PAF receptor gene, and Raji and Wa which are B cells.

**13** The monoclonal antibody according to Claim 1, which reacts with a peptide of the extracellular third domain of the PAF receptor, belongs to a subclass of IgG2a, has a κ-L chain, and reacts with proteins of about 40 kDa in CHO-PR1 and CHO-WT6'A which are cells transfected with a PAF receptor gene, and Raji and Wa which are B cells.

**14.** An active fragment selected from the group consisting of F(ab')₂, Fab', Fab, Fv and recombinant Fv of the monoclonal antibody according to Claim 1.

**15.** A process for producing monoclonal antibodies specifically reacting with a PAF receptor, which comprises the steps of:
(1) immunizing a rodent with a peptide of a PAF receptor,
(2) taking out the spleen of the immune rodent to form a suspension of splenocytes,
(3) mixing the suspension of the splenocytes with myeloma cells of a mouse in a hybridization accelerator to fuse both cells,
(4) diluting the fused cells with a medium which does not favor unfused myeloma cells to culture the fused cells, thereby sorting hybridomas produced by the fusion of the antibody-producing cells with the myeloma cells,
(5) confirming the presence of an antibody in a supernatant in each of culture wells separately containing the hybridomas using, as an indicator, the reactivity to the PAF receptor peptide,
(6) selecting hybridomas which produce the desired antibodies and then cloning the hybridomas by the limiting dilution technique, and
(7) recovering the desired antibodies from the culture supernatants of the monoclonal hybridoma cells.

**16.** The production process according to Claim 15, wherein the rodent is a Balb/c mouse, and the myeloma cell is P3U1.

**17.** The production process according to Claim 15, wherein the PAF receptor peptide is a peptide of the extracellular third or forth domain, or the intracellular fourth domain of the PAF receptor.

**18.** Hybridomas separately producing monoclonal antibodies which react specifically with a PAF receptor.

**19.** The hybridomas according to Claim 18, which are obtained by the production process according to Claim 15.
